Europäisches Patentamt

European Patent Office

Office européen des brevets

(11) Numéro de publication: **0 606 039 A1**

# (12) DEMANDE DE BREVET EUROPEEN

(21) Numéro de dépôt: **93480070.7**

(51) Int. Cl.⁵: **A61K 35/78**

(22) Date de dépôt: **14.06.93**

(30) Priorité: **08.01.93 FR 9300324**

(43) Date de publication de la demande:
**13.07.94 Bulletin 94/28**

(84) Etats contractants désignés:
**AT BE CH DE DK ES FR GB GR IE IT LI LU NL PT SE**

(71) Demandeur: **Perez, Adrien Guy
"Les Hippocampes",
Avenue Préconil
F-83120 Sainte Maxime(FR)**

(72) Inventeur: **Perez, Adrien Guy
"Les Hippocampes",
Avenue Préconil
F-83120 Sainte Maxime(FR)**

(54) **Composition de sève et d'extrait végétal.**

(57) L'objet de l'invention est relatif à une association de deux produits naturels, l'un étant pris systématiquement pour base et dans la plus importante : c'est la sève de plante, d'arbres ou de vignes, l'autre comme complément, qui est un extrait végétal ou une association d'extraits végétaux obtenus par diverses techniques, par exemple : huiles essentielles, alcoolatures, alcoolats, teintures, hydrolats, macérats. L'ensemble associé créant une synergie dans l'obtention d'actions thérapeutiques dues aux caractéristiques biochimiques de chacun d'eux.

EP 0 606 039 A1

PREAMBULE

L'objet de la présente est l'extension des formulations précédemment décrites sous le numéro d'enregistrement national 92 OO378, déposé le 10 janvier 1992, à l'utilisation de sèves végétales autres que la sève de vigne.

Les précédentes compositions, associations de plusieurs produits naturels complémentaires, agissant en synergie d'un point de vue thérapeutique, ont fait l'objet d'études complémentaires visant à déterminer.

- Les propriétés physico-chimiques
- La composition chimique minérale
- La composition chimique organique
- La composition bactériologique
- La stabilité dans le temps
- Les propriétés thérapeutiques

La présente invention permet ainsi, en associant systématiquement une sève végétale (sève de vigne, sève d'arbre, sève de plantes) ou tout autre extrait végétal acqueux, avec un extrait végétal organique naturel (alcoolature, huile essentielle, cennoïde, sève, teinture, intrait, phytols,...), d'obtenir une composition stable dans le temps, de composition connue, contenant les éléments minéraux de la base (sève végétale) et les éléments organiques des compléments (extraits végétaux organiques).

I - PROPRIETES PHYSICO-CHIMIQUES

La base commune à toutes les compositions objets de la présente sont des émulsions stables pour plusieurs mois, qu'il suffit d'agiter pendant quelques secondes, lorsque l'émulsion est "cassée", notamment si celle-ci est stockée à une température inférieure à 10°.

La sève végétale, base commune à toutes les compositions objet de la présente, apparait sous la forme d'une solution aqueuse, présentant un léger trouble résultant de la dissolution partielle de quelques composés organiques, mais surtout de nombreux éléments minéraux.

Après adjonction des produits naturels végétaux, comme les huiles essentielles par exemple, les compositions se présentent sous la forme d'émulsions stables pendant plusieurs mois, qui peuvent ensuite décanter pour apparaître ensuite sous la forme de phases distinctes, une phase inférieure, aqueuse, une phase supérieure organique. Les analyses physico-chimiques effectuées sur les compositions ont données les résultats suivants:

Densité 0,825 < d25 < 0,985 selon la composition analysée

Indice de réfraction 1,306 < IR < 1,512 selon la composition analysée

Les pouvoirs rotatoires, $\alpha O$, varient en fonction des huiles essentielles et de leurs proportions relatives. Les pouvoirs rotatoires ne peuvent être mesurés que sur le mélange des huiles essentielles, avant leur adjonction aux sèves végétales. Ils sont dans tous les cas conformes aux normes concernant les produits naturels utilisés dans l'industrie des parfums, cosmétiques et aromes alimentaires.

II - COMPOSITION CHIMIQUE MINERALE

Afin de vérifier la composition minérale des compositions, objet de la présente, des analyses ont été effectuées, par Spectrométrie d'émission, Spectrométrie d'absorption et chromatographie liquide haute performance ionique.

Les analyses ont pour objet de vérifier la teneur exacte en oligo-éléments d'origine minérale, responsables de l'activité thérapeutique des compositions décrites.

Appareillage :    spectrométre d'émission à plasma, système "sans reflux"
                  spectromètre d'absorption
                  balance analytique de précision 0,0001g

Produit:          acide nitrique Analypur
                  eau distillée millipure M.Q.

Protocole : peser environ exactement 5g dans une erlenmeyer à col codé, ajouter 10ml d'acide nitrique pur, ajuster les réfrigérants à boules et chauffer à reflux léger pendant 12h. Porter ensuite à ébullition lente pendant 2 heures, puis laisser refroidir après avoir rincé les réfrigérants avec 5ml d'eau, transférer dans une fiole de 25 ml et compléter au trait de jauge. Si la solution n'est pas limpide la filtrer.

Préparer une gamme étalon de 0, O.5 et 1mg/L en respectant la proportion d'acide nitrique (1O/25). Doser au spectromètre d'émission atomique en acceptant la calibration pour une erreur inférieure à 2%. Corriger la dérive éventuelle en cours d'analyse par le passage d'une solution standard en début et en fin de mesure.

Cas particulier de l'Iode :

Dosage par HPLC ionique et détection conductimétrique sur colonne anionique élément : phosphate de potassium 2mM, ph 4,5.

## III - COMPOSITION CHIMIQUE ORGANIQUE

Afin de vérifier la composition organique des formules citées dans la présente, des molécules organiques, acides aminés, acides organiques, sont analysés par chromatographie en phase gazeuse.

Appareillage : Chromatographe en phase gazeuse Colonne capillaire polaire, L = 50m 0 = 0,2mm Détecteur : Ionisation de flamme (composés connus) Spectromètre de masse (composés inconnus) Capture d'électrons (composés hologénés)

Conditions d'analyses : 60° pendant 5mn, puis gradien de 2°C/mn jusqu'à 220°C, puis isotherme ) 220°C pendant 15 minutes avant injection suivante.

Identification de composés : par étalonnage externe et temps de rétention pour les composés connus, et par spectres de masses pour les composés inconnus, tous les composés représentant plus de 5% en poids ont été identifiés selon ce protocole expérimental.

## IV - COMPOSITION BACTERIOLOGIQUE

Afin de vérifier la qualité bactériologique des compositions objets de la présente, toutes les analyses nécessaires à la formulation des produits à usage alimentaire ou thérapeutique ont été effectuées, par la méthode du nombre le plus probable, après mise en culture. C'est ainsi que sont dénombrés les germes suivants :

germes aérobies à 30°c selon la norme NF VO8O1 1 (XI 178)

coliformes à 30°c selon la norme NF VO8O15 (XI 178)

Coliformes fecaux selon la norme NF V08O17 (VI 18O)

Staphylococcus aureus selon la norme NF VO8O14(I 184)

Anaerobies sulfito-réductrices sur milieu TSN

Salmonella selon la norme ISO 6579 (VIII 190)

Moisissures et levures selon la norme (milieu FOGA) NF VO3454 (XII 181)

## V - STABILITE DANS LE TEMPS

Les même protocoles expérimentaux ont été utilisés pour les analyses de composition, et ceci sur une durée d'une année, après fabrication, mélange, traitement UV (bande 254mm) conditionnement en flacon teinté bleu ou marron.

## VI - EXEMPLES DE COMPOSITIONS ET PROPRIETES THERAPEUTIQUES

Les compositions ,conséquences de l'invention, sont testées en Laboratoire et font l'objet d'études chimiques depuis plusieurs mois, afin d'en contrôler l'efficacité; bien que leur formulation s'appuie systématiquement sur des résultats confirmés par la tradition et sur une bibliographie internationale très complète.

A titre d'exemples, des formules variantes de la composition objet de l'invention, à base de sèves végétales et d'extraits végétaux peuvent être les suivantes mais ne sont pas limitatives.

## VII - EXEMPLE DE COMPOSITION DE FORMULE

Les formules entrant dans le cadre de la présente invention ont été analysées selon les protocoles précédemment décrits. C'est ainsi qu'une formule à base de sève de vigne et d'huiles essentielles naturelles,dans les proportions suivantes :

sève de vigne 1OOOmg

huiles essentielles de

angelica archangelica 166mg

citrus reticulata 166mg

lippia citriodora 333mg

a donné les résultats suivants :

* analyses physico-chimiques : d25 0,887 ; Ir 1,458
* composition chimique minérale : (en ppm)

| | | | | |
|---|---|---|---|---|
| Fe | 1,5 | | I | <1 |
| Mn | < 0,5 | | K | 54 |
| Ca | 140 | | Na | 2 |
| Mg | 14 | | Ca | < 0,1 |
| L | 30 | | Hg | < 0,1 |
| Se | < 0,5 | | Pb | < 0,1 |
| Zn | < 0,1 | | As | < 0,1 |
| Cu | < 0,1 | | | |
| Cr | < 0,5 | | NO3 | < 1 mg/l |
| Mo | < 0,1 | | Cl | < 1 mg/l |
| Co | < 0,1 | | SO4 | < 1 mg/l |

recherches des résidus de pesticides (méthode multi-résidus)

Détecteur N.P.D.
Détecteur à captures d'électrons : pas de détection de résidus chlorés, bromés, fluorés révélateurs de pesticides.

* composition chimique organique

| | |
|---|---|
| limonène | ⎤ 52 % |
| a-pinène, b-pinène, g-terpinène camphène, terpinolène | ⎤ 3% |
| geraniol, citronellol | ⎤ 12,2% |

*composition bactériologique

| | |
|---|---|
| germes aérobies à 30°C | 1.680.000/g |
| coliformes à 30° | 160.000/g |
| coliformes fécaux | < 10/g |
| Staphylococcus curveus | < 100/g |
| Anaerobies sulfito-réducteurs | < 10/g |
| Salmonella | absence |
| moisissures | < 10/g |
| levures | < 10/g |

VII - CONCLUSIONS

Les différentes compositions objet de la présente, contiennent, après analyses poussées, tous les oligo-éléments minéraux et organiques nécessaires en tant qu'inducteurs de réactions biochimiques, ainsi que dans les composés organiques présents dans les huiles essentielles, dont les propriétés thérapeutiques sont actuellement reconnues. La stabilité de ces compositions, étudiée sur une durée de plusieurs mois, permet de conclure quant à l'efficacité de l'exposition aux radiations ultra-violettes, concernant la stérilisation.

Toute variante obtenue par adjonction d'extraits végétaux organique, à une base de sève végétale dans des proportions où la sève est le composé le plus important, est l'objet de la présente.

**Revendications**

1.  Produits adjuvants d'action thérapeutiques, selon les revendications décrites sous le numéro d'enregistrement national n° 92 OO378, dont l'un est une sève végétale, et l'autre un ou des extraits végétaux, l'association des composants agissant en synergie.

2.  Composition selon la revendication 1, caractérisée en ce qu'elle renferme de la sève végétale à l'état naturel, porteuse d'éléments minéraux et oligo-éléments, inducteurs de réactions biochimiques favorables à l'organisme humain et animal. Ladite sève, largement majoritaire, associée à des extraits végétaux tels que les huiles essentielles ou tout autre extrait organique, associés en quantité minoritaire, agit sur les systèmes physiologiques, ces extraits ayant des actions thérapeutiques traditionnellement connues.

3.  Compositions selon les revendications 1 et 2, qui peut agir au niveau externe de l'organisme, (par exemples non limitatifs, la repousse des poils ou des cheveux) mais aussi au niveau interne.

4.  Compositions selon les revendications 1, 2, 3, et 4 à base de sève végétale en quantité largement supérieure (par exemple non limitatifs la sève de vigne, la sève d'arbre, la sève de plantes), associée à des extraits végétaux (par exemples non limitatifs des huiles essentielles, expressions, alcoolatures) en quantité inférieure.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| D,E | FR-A-2 686 019 (A.G. PEREZ) <br> * le document en entier * <br> --- | 1-4 | A61K35/78 |
| X | DE-A-35 33 563 (M. BIRZER) <br> * le document en entier * <br> --- | 1-4 | |
| X | GB-A-2 124 903 (H. THOMM) <br> * le document en entier * <br> --- | 1-4 | |
| X | PATENT ABSTRACTS OF JAPAN <br> vol. 8, no. 77 (C-218) <br> & JP-A-59 001 406 (FUKUE ITOU) 6 Janvier 1984 <br> * abrégé * <br> --- | 1-4 | |
| X | PATENT ABSTRACTS OF JAPAN <br> vol. 8, no. 77 (C-218) <br> & JP-A-59 001 408 (FUKUE ITOU) 6 Janvier 1984 <br> * abrégé * <br> ----- | 1-4 | |

**DOMAINES TECHNIQUES RECHERCHES (Int.Cl.5)**

A61K

Le présent rapport a été établi pour toutes les revendications

| Lieu de la recherche | Date d'achèvement de la recherche | Examinateur |
|---|---|---|
| LA HAYE | 25 Avril 1994 | Orviz Diaz, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant